(19) Europäisches Patentamt
European Patent Office
Office européen des brevets

(11) Veröffentlichungsnummer : **0 080 194**
**B1**

(12) **EUROPÄISCHE PATENTSCHRIFT**

(45) Veröffentlichungstag der Patentschrift : **22.01.86**

(51) Int. Cl.[4] : **C 07 K 7/06**, A 61 K 37/02

(21) Anmeldenummer : **82110742.2**

(22) Anmeldetag : **20.11.82**

(54) **Neue Peptide und Verfahren zu ihrer Herstellung.**

(30) Priorität : **25.11.81 DE 3146598**

(43) Veröffentlichungstag der Anmeldung :
**01.06.83 Patentblatt 83/22**

(45) Bekanntmachung des Hinweises auf die Patenterteilung : **22.01.86 Patentblatt 86/04**

(84) Benannte Vertragsstaaten :
**AT BE CH DE FR GB IT LI NL SE**

(56) Entgegenhaltungen :
**EP-A- 0 025 897**
**EP-A- 0 037 246**
**GB-A- 1 596 535**
**CHEMICAL ABSTRACTS, Band 97, Nr. 7, 1982, Seite 694, Nr. 56235a, Columbus, Ohio, USA, M. ENGELHARD et al.: "Synthesis of retinylidene-peptides of bacteriorhodopsin from halobacterium halobium"**

(73) Patentinhaber : **HOECHST AKTIENGESELLSCHAFT**
**Postfach 80 03 20**
**D-6230 Frankfurt am Main 80 (DE)**

(72) Erfinder : **König, Wolfgang, Dr.**
**Eppsteiner Strasse 25**
**D-6238 Hofheim am Taunus (DE)**
Erfinder : **Geiger, Rolf, Prof. Dr.**
**Heinrich-Bleicher-Strasse 33**
**D-6000 Frankfurt am Main 50 (DE)**
Erfinder : **Obermeier, Rainer, Dr.**
**Langenhainer Weg 14**
**D-6234 Hattersheim am Main (DE)**
Erfinder : **Müllner, Hubert, Dr.**
**Pestalozzistrasse 4**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Aus Thymusextrakten wurden mehrere Peptide isoliert (z. B. Thymosin-$\alpha_1$ und Thymopoietin II), die zur Differenzierung (« Riefung ») thymusabhängiger Lymphocyten (T-Zellen) beitragen. Auch eine Teilsequenz von Thymopoietin II, Arg-Lys-Asp-Val-Tyr, zeigt in den entsprechenden Testsystemen, z. B. dem Rosettentest, eine thymopoietinartige Wirkung (Science (1979) *204*, 1309-1310).

Für die Untersuchung über den Einfluß kleinerer Peptide auf Lymphocyten eignet sich der genannte Rosettentest und auch der PHA-Stimulationstest.

Im Rosettentest wird die Reifung von Lymphozyten aus humanem Nabelschnurblut durch Rosettierung mit Schafserythrozyten in der Kälte verfolgt. Dieser Test ist spezifisch für T-Zellen, d. h. er gestattet die Beobachtung der Verschiebung von $R^-$-T-Zellen zu $R^+$-T-Zellen. Das Nabelschnurblut enthält relativ viele unreife oder nicht ausdifferenzierte Lymphozyten, so daß der Basiswert (Normalwert) an rosettenbildenden T-Zellen niedriger liegt als bei Lymphozyten des Peripherblutes Erwachsener.

Auf Grund von Untersuchungen des Blutes von Patienten, die an Autoimmunkrankheiten oder Tumoren erkrankt sind, läßt sich eine niedrige Rosettenzahl korrelieren mit einem niedrigen Immunstatus. Im Test werden die Rosettenzahlen von Lymphozyten-Erythrozyten-Systemen ausgezählt und verglichen, die entweder nur in einem für Nähr-Medium ohne Zusatz oder in dem Nähr-Medium unter Zusatz der zu prüfenden Substanz vorinkubiert wurden.

Die Konzentration der untersuchten Peptide wird in diesem Test zwischen etwa 10 ng/ml bis 1 µg/ml, 5 µg/ml, 10 µg/ml und 20 µg/ml variiert. In der Auswertung werden nur solche Rosetten erfaßt, die mindestens aus einem Lymphozyten und drei anhaftenden Erythrozyten bestehen.

Im PHA-Stimulationstest (PHA = Phythämagglutinin) oder auch Lymphozytentransformationstest werden Rückschlüsse über die Anzahl der reifen, d. h. stimulierbaren Lymphocyten nicht über eine Untersuchung der Oberflächenantigene gewonnen, sondern durch den Funktionstest auf Stimulierbarkeit mit dem Pflanzenlektin PHA. Die Lymphozyten (T-Zellen) werden durch das Lektin, ähnlich wie durch bakterielle oder virale Antigene zu einer Blastentransformation angeregt. Diese führt entweder direkt oder durch Ausschüttung von Lymphokinen zu einer Proliferation. Der Einbau von Radioaktivem Thymidin innerhalb einer bestimmten Zeit ist dann ein Maß für die Anzahl der stimulierten Zellen. Nur die reifen oder immunologisch potenten T-Zellen werden stimuliert. Es läßt sich daher der Einfluß einer Substanz für die Lymphozytenreifung mit diesem Test verfolgen. Allerdings muß die Stimulierung suboptimal sein, denn bei höheren Konzentrationen werden auch andere Subpopulationen von Lymphozyten stimuliert und der Effekt läßt sich nicht mehr beobachten. Die zu untersuchenden Peptide waren in verschiedenen Konzentrationen zum Kulturmedium gegeben worden. Sie sind während der gesamten Testdauer (bis zu 72 Stunden) präsent.

Ein möglicher Abbau der Peptide durch Serum oder Lymphozytenproteasen wird bei diesen Untersuchungen nicht berücksichtigt.

Es wurden nun Peptide gefunden, die insbesondere in dem spezifischen PHA-Test das bekannte Peptid Arg-Lys-Asp-Val-Tyr in der Wirkung übertreffen. Diese neuen Peptide können entsprechend den allgemeinen Eigenschaften ihrer Glieder wie folgt charakterisiert werden :

basisch-basisch-aromatisch-aromatisch-beliebig.

Die Erfindung betrifft demnach Peptide der allgemeinen Formel

$$B_1—B_2—A_1—A_2—X,$$

in welcher bedeuten :

$B_1$ Arginin oder Lysin, jeweils in der L- oder D-Konfiguration, wobei die $\alpha$-Aminogruppe gegebenenfalls acyliert ist,

$B_2$ Arginin oder Lysin,

$A_1$ Phenylalanin, Tyrosin oder Tryptophan, jeweils in der L- oder D-Konfiguration,

$A_2$ Phenylalanin, Tyrosin oder Tryptophan und

X Glutamin, Prolin, Glycin oder Arginin, wobei die Carboxylgruppe gegebenenfalls mit einem aliphatischen Alkohol mit 1-6 C-Atomen verestert ist,

sowie deren physiologisch verträglichen Salze.

Der Substituent (Schutzgruppe) an der $\alpha$-Aminogruppe von $B_1$ und das Glied X sind für die Wirkung der erfindungsgemäßen Peptide nicht kritisch. Sie können aber für die Stabilität und die Bioverfügbarkeit und somit in quantitativer Hinsicht von Bedeutung sein.

Besonders vorteilhaft sind in diesem Sinne Schutzgruppen vom Urethantyp, da sie die Peptide vor einem N-terminalen Abbau durch Acylasen und Aminopeptidasen schützen. Infrage kommen vor allem die in der Peptidchemie gebräuchlichen Urethanschutzgruppen, wie Benzyloxycarbonyl oder tert.-Butyloxycarbonyl. Aber auch einfache Alkyloxycarbonylgruppen erfüllen diesen Zweck. Weniger güngstig sind normale Acylgruppen, die wie Acetyl das Peptid zwar vor Aminopeptidasen, nicht aber vor Acylasen zu schützen vermögen.

Hinsichtlich der Wirksamkeit gibt es z. B. zwischen X = Gly, Arg oder Prolin nur quantitative Unterschiede. Als besonders wirksam erwiesen sich Peptide, in denen X eine basische Aminosäure, wie Arginin darstellt. Auch eine Veresterung der Carboxylgruppe mit einem aliphatischen Alkohol, wie

Methanol, Äthanol oder einem höheren Alkohol, z. B. n-Hexanol, kann für die Wirkung und für die Bioverfügbarkeit des Peptids von Vorteil sein.

Gegenstand der Erfindung ist ferner ein Verfahren zur Herstellung der genannten Peptide. Das Verfahren ist dadurch gekennzeichnet, daß man Aminosäuresequenzen der Formel

$$B_1—B_2—A_1—A_2—X$$

nach Methoden der Peptidchemie aufbaut und die erhaltenen Peptide gegebenenfalls in physiologisch verträgliche Salze überführt. Die Synthese der erfindungsgemäßen Verbindungen folgt den bekannten Methoden der peptidchemie, wie sie z. B. in Houben-Weyl, Methoden der Organischen Chemie, Band 15, ausführlich beschrieben sind. Die auf den folgenden Seiten wiedergegebenen Beispiele erläutern die an sich bekannten Syntheseverfahren.

Die erfindungsgemäßen Verbindungen können zur Behandlung von Immundefizienzen, viralen und fungoiden, sowie chronisch bakteriellen Infekten, Autoimmunkrankheiten, sowie zur Therapie von Erkrankungen dienen, die durch Zellen mit immunologisch relevanten Veränderungen der Zellmembranmerkmale (z. B. Tumorzellen) verursacht werden.

In diesem Sinne ist Gegenstand der Erfindung auch die Verwendung der genannten Peptide ganz allgemein zur Beeinflussung der Reifung von T-Lymphozyten, sowie ein Mittel, das die Peptide als Wirkstoff enthält.

Die Anwendung der erfindungsgemäßen Peptide kann intravenös, subcutan oder intranasal erfolgen.

Die Dosierung bei parenteraler Gabe liegt bei 0.1-50 µg, bei intranasaler Gabe bei 1-500 µg je Einzeldosis. Sie kann in schweren Fällen auch erhöht werden, da toxische Eigenschaften bisher nicht beobachtet wurden. Auch eine Herabsetzung der Dosis ist möglich.

Die erfindungsgemäßen Verbindungen können intranasal oder parenteral in entsprechender pharmazeutischer Zubereitung verabreicht werden. Für eine intranasale Anwendungsform werden die aktiven Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsionen gebracht. Als Lösungsmittel für die neuen aktiven Verbindungen und die entsprechenden physiologisch verträglichen Salze kommen z. B. in Frage : Wasser, physiologische Kochsalzlösungen oder Alkohole, z. B. Äthanol, Propandiol oder Glycerin, daneben auch Zuckerlösungen wie Glukose- oder Mannitlösungen, oder auch eine Mischung aus den verschiedenen genannten Lösungsmitteln.

Die Wirksamkeit der Verbindungen wird durch nachfolgende pharmakologische Daten belegt.

Folgende Verbindungen wurden im PHA-Test untersucht

(A) Lys-Arg-Tyr-Tyr-Gly-OEt.

(B) Lys-Lys-Tyr-Phe-Arg-OH

(D) D-Lys-Arg-D-Phe-Trp-Pro-OH

(E) Arg-Lys-Tyr-Phe-Gln-OH

Als bekannte Vergleichssubstanz diente die Verbindung

(V) Arg-Lys-Asp-Val-Tyr-OH

Ergebnisse des PHA-Stimulationstests :

| Prüfsubstanz | Konzentration µg/ml | PHA-Konzentration µg/ml | Einbau von $^3$H-Thymidin (cpm) | Stimulationsindex | $\bar{X} \pm SEM$ |
|---|---|---|---|---|---|
| V | 1 | 10 | 59073 auf 61624 | 1.06 | |
| | | 8 | 46392 auf 58607 | 1.26 | 1.17 ± 0.06 |
| | | 4 | 26875 auf 32247 | 1.20 | |
| A | 1 | 10 | 14356 auf 18388 | 1.28 | |
| | | 8 | 11517 auf 14031 | 1.22 | 1.32 ± 0.07 |
| | | 4 | 2727 auf 3947 | 1.45 | |

(Fortsetzung)

| Prüf-substanz | Konzentration µg/ml | PHA-Konzen-tration µg/ml | Einbau von $^3$H-Thymidin (cpm) | Stimula-tions-index | $\bar{x}$ + SEM |
|---|---|---|---|---|---|
| B | 1 | 10 | 19848 auf 25888 | 1.30 | 1.16 ± 0.05 |
| | | 8 | 5300 auf 5801 | 1.09 | |
| | | 4 | 5499 auf 6073 | 1.10 | |
| D | 1 | 10 | 5038 auf 8713 | 1.73 | 1.33 ± 0.2 |
| | | 8 | 5185 auf 5924 | 1.14 | |
| | | 4 | 4415 auf 4999 | 1.13 | |
| E | 1 | 10 | 12650 auf 15231 | 1.20 | 1.12 ± 0.05 |
| | | 8 | 12693 auf 13148 | 1.04 | |
| | | 4 | 8105 auf 9072 | 1.12 | |

Die folgenden Beispiele veranschaulichen die Erfindung, insbesondere die Synthese der erfindungsgemäßen Peptide.

Es werden in der Regel die nach IUPAC verwendeten Abkürzungen benutzt.

Boc tert.-Butyloxycarbonyl
Bu$^t$ tert.-Butyl
DCC Dicyclohexylcarbodiimid
Et Äthyl
HOBt 1-Hydroxybenzotriazol
HOObt 3-Hydroxy-4-oxo-3.4-dihydro-1.2.3-benzotriazin
Mbh 4,4'-Dimethoxybenzhydryl
Me Methyl
Z Benzyloxycarbonyl

## Beispiel 1

H-Lys-Arg-Tyr-Tyr-Gly-OEt-diacetat

a) Z-Tyr(Bu$^t$)-Gly-OEt

Zu einer Lösung von 18.55 g (50 mmol) Z-Tyr(Bu$^t$)-OH, 6.98 g (50 mmol) H-Gly-OEt · HCl und 6.75 g (50 mmol) HOBt in 75 ml Dimethylformamid gibt man unter Rühren bei 0 °C 6.4 ml (50 mmol) N-Äthylmorpholin und 11.33 g (55 mmol) DCC. Man läßt zwei Stunden bei 0 °C rühren und über Nacht bei Raumtemperatur stehen. Der Niederschlag wird abgesaugt und das Filtrat im Hochvakuum eingeengt. Der Rückstand wird zwischen Essigester und Wasser verteilt. Die Essigesterphase wird mit $NaHCO_3$-Lösung, $KHSO_4/K_2SO_4$-Puffer und Wasser ausgeschüttelt, über $Na_2SO_4$ getrocknet und eingeengt. Der Rückstand wird in Diisopropyläther gelöst. Von ungelöstem Material wird abfiltriert und das Filtrat mit Petroläther versetzt. Man kühlt einige Stunden im Eisbad und saugt den Niederschlag ab. Ausb. 20.63 g (89 %), Schmp. 108 °C, $[\alpha]_D^{22}$ = − 13.4° (c = 1, Methanol).

b) H-Tyr(Bu$^t$)-Gly-OEt · HCl

19.78 g (43 mmol) Z-Tyr(Bu$^t$)-Gly-OEt werden in 200 ml Methanol gelöst. Dazu gibt man Pd/Kohle-Katalysator und leitet unter Rühren und unter Zugabe von ca. 2N methanolischer HCl bei pH 4.5 (Autotitrator) Wasserstoff durch die Lösung, bis keine methanolische HCl mehr aufgenommen wird. Danach wird der Katalysator abgesaugt und das Filtrat eingeengt. Der Rückstand wird mit Äther verrührt und abgesaugt. Ausb. 14.87 g (93.7 %), Schmp. 140-142°, $[\alpha]_D^{22}$ = + 30.9° (c = 1, Methanol).

c) Z-Tyr(Bu$^t$)-Tyr(Bu$^t$)-Gly-OEt

Zu einer Lösung von 14.35 g (40 mmol) H-Tyr(Bu$^t$)-Gly-OEt · HCl, 14,84 g (40 mmol) Z-Tyr(Bu$^t$)-OH und 5.4 g (40 mmol) HOBt in 100 ml Dimethylformamid gibt man bei 0 °C 5.12 ml (40 mmol) N-Äthylmorpholin und 8.65 g (42 mmol) DCC. Man arbeitet wie bei Beispiel 1 a auf. Der Rückstand wird mit Petroläther verrieben. Ausb. 26.3 g. Zur Reinigung wird die Substanz in Methylenchlorid/Aceton (9 : 1) über 150 g Kieselgel chromatographiert. Die Fraktionen wurden eingeengt und der Rückstand mit Äther verrieben. Ausb. 19.74 g (73 %), Schmp. 113°, $[\alpha]_D^{22} = -28.7°$ (c = 1, Methanol).

d) H-Tyr(Bu$^t$)-Tyr(Bu$^t$)-Gly-OEt · HCl

18.9 g (28 mmol) Z-Tyr(Bu$^t$)-Tyr(Bu$^t$)-Gly-OEt werden in 200 ml Methanol gelöst und analog Beispiel 1 b katalytisch hydriert. Der Rückstand wird mit einer Mischung von 250 ml Äther und 250 ml Petroläther verrieben und abgesaugt. Ausb. 15.65 g (96 %) · $[\alpha]_D^{22} = +7.7°$ (c = 1, Methanol).

e) Z-Arg($Z_2$)-Tyr(Bu$^t$)-Tyr(Bu$^t$)-Gly-OEt

Zu einer Lösung von 5.78 g (10 mmol) H-Tyr(Bu$^t$)-Tyr(Bu$^t$)-Gly-OEt · HCl und 1.35 g (10 mmol) HOBt in 30 ml Dimethylformamid gibt man 1.28 ml (10 mmol) N-Äthylmorpholin und 8.15 g (10.5 mmol) Z-Arg ($Z_2$)-OTcp. Man läßt 4 Stunden bei Raumtemperatur rühren, läßt über Nacht bei Raumtemperatur stehen und verrührt die entstandene gallertige Masse mit 250 ml Wasser. Der Niederschlag wird abgesaugt und nacheinander mit $KHSO_4/K_2SO_4$-Puffer, $NaHCO_3$-Lösung und Wasser verrührt und abgesaugt. Ausb. 10.47 g (95 %). Schmp. 163-165°, $[\alpha]_D^{23} = -16.1°$ (c = 1, Dimethylformamid).

f) H-Arg-Tyr(Bu$^t$)-Tyr(Bu$^t$)-Gly-OEt · 2 HCl

9.9 g (9 mmol) Z-Arg($Z_2$)-Tyr(Bu$^t$)-Tyr(Bu$^t$)-Gly-OEt werden in 300 ml Methanol suspendiert und analog Beispiel 1 b katalytisch hydriert. Der Rückstand wird mit Äther verrieben. Ausb. 6.22 g (92 %). Schmp. 76-83°, $[\alpha]_D^{23} = +16.6°$ (c = 1, Methanol).

g) Z-Lys(Boc)-Arg-Tyr(Bu$^t$)-Tyr(Bu$^t$)-Gly-OEt

Zu einer Lösung von 5.98 g (8 mmol) H-Arg-Tyr(Bu$^t$)-Tyr(Bu$^t$)-Gly-OEt · 2 HCl und 1.08 g HOBt (8 mmol) in 40 ml Dimethylformamid gibt man 1.03 g N-Äthylmorpholin und 4.76 g (8.5 mmol) Z-Lys(Boc)-OTcp. Man läßt 6 Stunden bei Raumtemperatur rühren und über Nacht bei Raumtemperatur stehen. Man arbeitete analog Beispiel 1 a auf ohne jedoch sauer auszuschütteln. Der Rückstand wird mit Äther verrieben und zur weiteren Reinigung in Methylenchlorid/Methanol (9 : 1) an 100 g Kieselgel chromatographiert. Die entsprechenden Fraktionen werden eingeengt und der Rückstand mit Äther verrieben. Ausb. 5.88 g (72 %), Schmp. 96-99°, $[\alpha]_D^{25} = -22.0°$ (c = 1, Dimethylformamid).

h) H-Lys-Arg-Tyr-Tyr-Gly-OEt-diacetat

5.0 g (5 mmol) Z-Lys(Boc)-Arg-Tyr(Bu$^t$)-Tyr(Bu$^t$)-Gly-OEt werden in 50 ml 90-proz. Trifluoressigsäure gelöst. Man läßt zwei Stunden bei Raumtemperatur stehen und engt ein. Die Substanz wird zunächst mit Äther verrieben und abgesaugt. Danach wird mit Wasser verrieben und abgesaugt.

Anschließend wird die Substanz (= Z-Lys-Arg-Tyr-Tyr-Gly-OEt-trifluoracetat) in 60 ml 90-prozentige Essigsäure gelöst und nach Zugabe von Pd/Kohl-Katalysator unter Durchleiten von Wasserstoff katalytisch hydriert. Nach beendigter Hydrierung wird vom Katalysator abgesaugt und das Filtrat eingeengt. Der Rückstand wird in Wasser an einem schwach basischen Ionenaustauscher (Acetat-Form) chromatographiert. Das Eluat wird gefriergetrocknet. Ausb. 3.365 g. Zur Reinigung werden davon 800 mg in 90-proz. Methanol an einem vernetzen hydroxypropylierten Dextrangel chromatographiert. Ausb. 506 mg, $[\alpha]_D^{24} = -10.7°$ (c = 1, Methanol). Aminosäureanalyse (Hydrolyse in 6N HCl, 24 Stunden bei 120°) : Gly 1.0, Tyr 1.7, Lys 1.0, Arg. 0.9.

Beispiel 2

Z-Lys-Lys-Tyr-Phe-Arg-OH-diacetat

a) Z-Tyr(Bu$^t$)-Phe-OMe

Zu einer Lösung von 40.7 g (0.1 mmol) Z-Tyr(Bu$^t$)-OH, 21.6 g H-Phe-OMe-Hydrochlorid und 13.5 g (0.1 mmol) HOBt in 200 ml Dimethylformamid gibt man bei 0 °C 13 ml N-Äthylmorpholin und 22 g DCC. Man arbeitet wie bei Beispiel 1a auf. Der Rückstand wird mit Petroläther verrieben und abgesaugt. Ausb. 50.3 g (94 %), Schmp. 103-104°, $[\alpha]_D^{24} = -12.3°$ (c = 1, Methanol)

b) H-Tyr(Bu$^t$)-Phe-OMe · HCl

49.5 g Z-Tyr($Bu^t$)-Phe-OMe werden in 800 ml Methanol gelöst und analog Beispiel 1b katalytisch hydriert. Der Rückstand läßt sich nicht kristallisieren. Ausb. 43 g Öl.

c) Z-Lys(Boc)-Tyr($Bu^t$)-Phe-OMe

Zu einer Lösung von 21.5 g H-Tyr($Bu^t$)-Phe-OMe · HCl und 6.67 g HOBt in 150 ml Dimethylformamid gibt man 6.43 ml N-Äthylmorpholin und 27.67 g Z-Lys(Boc)-OTcp. Man rührt 3 Stunden bei Raumtemperatur und engt ein. Der Rückstand wird analog Beispiel 1a aufgearbeitet. Die Substanz wird aus Essigester/Petroläther kristallisiert. Ausb. 29.1 g (77 %), Schmp. 93-94°, $[\alpha]_D^{24} = -19.2°$ (c = 1, Methanol).

d) H-Lys(Boc)-Tyr($Bu^t$)-Phe-OMe · HCl

28 g Z-Lys(Boc)-Tyr($Bu^t$)-Phe-OMe werden in 300 ml Methanol gelöst und analog Beispiel 1b katalytisch hydriert. Der Rückstand kristallisiert aus Äther. Ausb. 22.4 g (91.8 %), Schmp. 145-147°, $[\alpha]_D^{24} = +18.2°$ (c = 1, Methanol).

e) Z-Lys(Boc)-Lys(Boc)-Tyr($Bu^t$)-Phe-OMe

Zu einer Lösung von 21.9 g H-Lys(Boc)-Tyr($Bu^t$)-Phe-OMe · HCl, und 4.46 g HOBt in 130 ml Dimethylformamid gibt man 4.3 ml N-Äthyl-morpholin und 18.48 g Z-Lys(Boc)-OTcp. Man läßt drei Stunden bei Raumtemperatur rühren. Danach fällt man mit 400 ml Wasser und 35 ml gesättigter $NaHCO_3$-Lösung das Peptid aus. Der Niederschlag wird abgesaugt. Aus Essigester/Petroläther wird kristallisiert. Ausb. 30.4 g (93 %), Schmp. 159-160°, $[\alpha]_D^{28} = -17.5°$ (c = 1, Methanol).

f) Z-Lys(Boc)-Lys(Boc)-Tyr($Bu^t$)-Phe-OH

20 g (ca. 20 mmol) Z-Lys(Boc)-Lys(Boc)-Tyr($Bu^t$)-Phe-OMe werden in 100 ml Dioxan fast vollständig gelöst. Dazu gibt man 20 ml 1N NaOH und rührt eine Stunde bei Raumtemperatur. Anschließend wird von Unlöslichem filtriert und das Filtrat mit 500 ml Eis-Wasser und 20 ml 1N HCl versetzt. Der Niederschlag wird abgesaugt und getrocknet. Ausb. 18.5 g. Laut DC in Methylenchlorid/Aceton (8 : 2) war die Verseifung nicht vollständig verlaufen. Die Substanz enthält noch größere Mengen Ausgangsprodukt. Durch fraktionierte Kristallisation aus Essigester wurden etwa 3 g reines Verseifungsprodukt aus dem Gemisch isoliert. Schmp. 156-158°, $[\alpha]_D^{25} = -13.1°$ (c = 1, Methanol).

g) Z-Lys(Boc)-Lys(Boc)-Tyr($Bu^t$)-Phe-Arg-$OBu^t$

Zu einer Lösung von 1.95 g (2 mmol) Z-Lys(Boc)-Lys(Boc)-Tyr($Bu^t$)-Phe-OH, 606 mg (2 mmol) H-Arg-$OBu^t$ · 2 HCl und 326 mg (2 mmol) HOObt in 5 ml Dimethylacetamid gibt man bei 0 °C 0.26 ml N-Äthylmorpholin und 440 mg DCC. Man läßt zunächst zwei Stunden bei 0 °C rühren und läßt über Nach bei Raumtemperatur stehen. Anderntags wird der Niederschlag abgesaugt und das Filtrat eingeengt. Ausb. 2.5 g. Zur Reinigung wird die Substanz zwei mal aus Essigester umkristallisiert. Ausb. 1.7 g, Schmp. 134-137°, $[\alpha]_D^{26} = -26.4°$ (c = 1, Methanol).

h) Z-Lys-Lys-Tyr-Phe-Arg-OH-diacetat

1.6 g Z-Lys(Boc)-Lys(Boc)-Tyr($Bu^t$)-Phe-Arg-$OBu^t$ werden in 15 ml 90-proz. Trifluoressigsäure gelöst. Man läßt eine Stunde bei Raumtemperatur stehen und engt ein. Der Rückstand wird mit Äther verrieben und abgesaugt. Anschließend wird der Rückstand in Wasser gelöst und über einen schwach basischen Ionenaustauscher (Acetat-Form) chromatographiert. Das Eluat wird eingeengt und in 90-proz. Methanol an einem vernetzten hydroxypropylierten Dextrangel chromatographiert. Ausb. 920 mg, $[\alpha]_D^{24} = -29.7°$ (c = 1, Methanol).

## Beispiel 3

H-Lys-Lys-Tyr-Phe-Arg-OH-diacetat

500 mg Z-Lys-Lys-Tyr-Phe-Arg-diacetat werden in 20 ml 90-proz. Essigsäure gelöst und analog Beispiel 1 h katalytisch hydriert. Der Rückstand wird in 90-proz. Methanol an einem vernetzten hydroxypropylierten Dextrangel chromatographiert. Das Peptid-haltige Eluat wird eingeengt, der Rückstand in Wasser gelöst und gefriergetrocknet. Ausb. 244 mg. Aminosäureanalyse (Hydrolyse in 6N HCl, 24 Stunden bei 120 °C) : Tyr 0.93, Phe 1.0, Lys 2.06, Arg 0.9.

## Beispiel 4

Z-D-Lys-Arg-D-Phe-Trp-Pro-OH

a) Z-Trp-Pro-OBu$^t$

Zu einer Lösung von 16.9 g (50 mmol) Z-Trp-OH, 8.55 g (50 mmol) H-Pro-OBu$^t$ und 6.75 g (50 mmol) HOBt in 75 ml Dimethylformamid gibt man bei 0 °C 10.92 g (53 mmol) DCC. Man läßt zwei Stunden bei 0 °C rühren und über Nacht bei Raumtemperatur stehen. Der Niederschlag wird abgesaugt und das Filtrat eingeengt. Der Rückstand wird analog Beispiel 1a aufgearbeitet. Der Rückstand wird mit Äther verrieben. Ausb. 19.49 g (79 %), Schmp. 173°, $[\alpha]_D^{25}$ = – 54.2° (c = 1, Dimethylformamid)

b) H-Trp-Pro-OBu$^t$ · HCl

16.64 g (40 mmol) Z-Trp-Pro-OBu$^t$ werden in 200 ml Methanol analog Beispiel 1 b katalytisch hydriert. Der Rückstand wird mit Äther verrieben. Ausb. 14.37 g, Schmp. 190-195°, $[\alpha]_D^{25}$ = – 22.4° (c = 1, Methanol).

c) Z-d-Phe-Trp-Pro-OBu$^t$

Zu einer Lösung von 3.46 g (10 mmol) H-Trp-Pro-OBu$^t$ · HCl, 2.99 g (10 mmol) Z-D-Phe-OH und 1.35 g (10 mmol) HOBt in 25 ml Dimethylformamid gibt man bei 0 °C 1.28 ml (10 mml) N-Äthylmorpholin und 2.26 g (11 mmol) DCC. Man läßt zwei Stunden bei 0 °C rühren und läßt über Nacht bei Raumtemperatur stehen. Der Niederschlag wird abgesaugt und das Filtrat eingeengt. Der Rückstand wird analog Beispiel 1a aufgearbeitet. Der Rückstand kristallisiert aus Äther. Ausb. 3.91 g (61 %), Schmp. 186°, $[\alpha]_D^{23}$ = – 53.6° (c = 1, Methanol).

Aus der Mutterlauge kann durch Zusatz von Petroläther noch Peptid ausgefällt werden. Ausb. 0.85 g, Schmp. 183°.

d) H-D-Phe-Trp-Pro-OBu$^t$ · HCl

3.7 g Z-D-Phe-Trp-Pro-OBu$^t$ werden in 100 ml Methanol analog Beispiel 1b katalytisch hydriert. Der Rückstand wird mit Äther verrieben. Ausb. 2.67 g, Schmp. 155-165°, $[\alpha]_D^{23}$ = – 75.8° (c = 1, Methanol).

e) Z-Arg($Z_2$)-D-Phe-Trp-Pro-OBu$^t$

Zu einer Lösung von 2.43 g (4.5 mmol) H-D-Phe-Trp-Pro-OBu$^t$ · HCl und 0.61 g (4.5 mmol) HOBt in 20 ml Dimethylformamid gibt man bei 0 °C 0.58 ml (4.5 mmol) N-Äthylmorpholin und 3.5 g (4.6 mmol) Z-Arg($Z_2$)-OTcp. Man läßt 6 Stunden bei Raumtemperatur rühren und über Nacht bei Raumtemperatur stehen. Der Ansatz wird eingeengt und analog Beispiel 1a aufgearbeitet. Der Rückstand wird mit Äther verrieben. Ausb. 4.01 g. Umkristallisation aus 25 ml 94 %igem Alkohol gibt 3.58 g (75 %) Schmp. 142-145°, $[\alpha]_D^{23}$ = – 19.2° (c = 1, Methanol)

f) H-Arg-D-Phe-Trp-Pro-OBu$^t$ · 2 HCl

3 g Z-Arg($Z_2$)-D-Phe-Trp-Pro-OBu$^t$ werden in 100 ml Methanol suspendiert und analog Beispiel 1b katalytisch hydriert. Der Rückstand wird mit Äther verrieben. Ausb. 2.01 g (97 %) Schmp. 190-200°, $[\alpha]_D^{23}$ = – 27.0° (c = 1, Methanol).

g) Z-D-Lys(Boc)-Arg-D-Phe-Trp-Pro-OBu$^t$

Zu einer Lösung von 1.47 g (2 mmol) H-Arg-D-Phe-Trp-Pro-OBu$^t$ · 2HCl und 0.27 g (2 mmol) HOBt in 10 ml Dimethylformamid gibt man bei 0 °C 0.26 ml (2 mmol) N-Äthylmorpholin und 1.23 g (2,2 mmol) Z-D-Lys(Boc)-OTcp. Man läßt 4 Stunden bei Raumtemperatur rühren, engt ein und verteilt den Rückstand zwischen Essigester und einer gesättigten $NaHCO_3$-Lösung. Die Essigesterphase wird mit Wasser ausgeschüttelt, über $Na_2SO_4$ getrocknet und eingeengt. Der Rückstand wird mit Äther verrieben und abgesaugt. Ausb. 1.65 g, Schmp. 125-132°, $[\alpha]_D^{25}$ = – 21.9° (c = 1, Methanol)

h) Z-D-Lys-Arg-D-Phe-Trp-Pro-OH

1.55 g Z-D-Lys(Boc)-Arg-D-Phe-Trp-Pro-OBu$^t$ werden in 14.5 ml 90-proz. Trifluoressigsäure und 1.5 ml Äthylmerkaptan gelöst. Man läßt eine Stunde bei Raumtemperatur stehen und engt ein. Der Rückstand wird in Wasser gelöst und über einen schwach basischen Ionenaustauscher (Acetat Form) chromatographiert. Das Eluat wird gefriegetrocknet. Ausb. 883 mg. Zur Reinigung wird das Peptid in 90-proz. Methanol an einem vernetzten hydroxypropylierten Dextrangel chromatographiert. Ausb. 570 mg, $[\alpha]_D^{22}$ = – 24.6° (c = 1, Methanol).

Beispiel 5

H-D-Lys-Arg-D-Phe-Trp-Pro-OH-triacetat

380 mg Z-D-Lys-Arg-D-Phe-Trp-Pro-OH-acetat werden in 20 ml 90-proz. Essigsäure gelöst und analog Beispiel 1 h katalytisch hydriert. Der Rückstand wird in 90-proz. Methanol an einem vernetzten hydroxypropylierten Dextrangel chromatographiert. Ausb. 140 mg, $[\alpha]_D^{23} = -26.8°$ (c = 1, Methanol).

Aminosäureanalyse (Hydrolyse in 6N HCl, 24 Stunden bei 120°) Pro 0.95, Phe 1.0, Lys 1.03, Arg 0.93, Trp wird bei der Hydrolyse zerstört, konnte aber durch ein UV-Spektrum nachgewiesen werden.

Beispiel 6

H-Arg-Lys-Tyr-Phe-Gln-OH-acetat

a) H-Tyr($Bu^t$)-Phe-Gln(Mbh)-OH-acetat

30 g Z-Tyr($Bu^t$)-Phe-Gln(Mbh)-OH werden in 600 ml 90-proz. Essigsäure gelöst und analog Beispiel 1h katalytisch hydriert. Der Rückstand wird mit Äther verrieben. Ausb. 24.2 g (88 %) Schmp. 192-193°, $[\alpha]_D^{24} = +16.9°$ (c = 1,80-proz. Essigsäure).

b) Z-Lys(Boc)-Tyr($Bu^t$)-Phe-Gln(Mbh)-OH

Zu einer Lösung von 5.6 g H-Tyr($Bu^t$)-Phe-Gln(Mbh)-OH-acetat und 0.95 g HOBt in 20 ml Dimethylformamid gibt man 3.92 g Z-Lys(Boc)-OTcp. Man rührt 4 Stunden bei Raumtemperatur und läßt über Nacht bei Raumtemperatur stehen. Anderntags wird der Ansatz mit 100 ml Wasser und 7 ml ges. $NaHCO_3$-Lösung verrührt. Der Niederschlag wird abgesaugt und getrocknet. Ausb. 8.09 g (95 %), Schmp. 177-184°, $[\alpha]_D^{28} = -10.6°$ (c = 1, 80-proz. Essigsäure).

c) H-Lys(Boc)-Tyr($Bu^t$)-Phe-Gln(Mbh)-OH-acetat

7.5 g Z-Lys(Boc)-Tyr($Bu^t$)-Phe-Gln(Mbh)-OH werden in 350 ml 90-proz. Essigsäure gelöst und analog Beispiel 1h katalytisch hydriert. Der Rückstand wird mit Äther verrieben und abgesaugt. Ausb. 4.81 g (74 %), Schmp. 136-138°, $[\alpha]_D^{28} = +8.2°$ (c = 1, 80-proz. Essigsäure).

d) Z-Arg($Z_2$)-Lys(Boc)-Tyr($Bu^t$)-Phe-Gln(Mbh)-OH

Zu einer Lösung von 2.57 g (2.5 mmol) H-Lys(Boc)-Tyr($Bu^t$)-Phe-Gln(Mbh)-acetat und 0.34 g HOBt in 10 ml Dimethylformamid gibt man eine Lösung von 1.9 g Z-Arg($Z_2$)-OTcp in 5 ml Dimethylformamid. Man rührt 5 Stunden bei Raumtemperatur und läßt über Nacht stehen. Danach verrührt man den Ansatz mit 50 ml Wasser und 3.5 ml gesättigter $NaHCO_3$-Lösung. Der Niederschlag wird abgesaugt, gut gewaschen und getrocknet. Ausb. 3.75 g (98 %), Schmp. 132-140° $[\alpha]_D^{24} = -0.19°$ (c = 1, Essigsäure).

e) Z-Arg ($Z_2$)-Lys-Tyr-Phe-Gln-OH

3 g (2 mmol) Z-Arg($Z_2$)-Lys(Boc)-Tyr($Bu^t$)-Phe-Gln(Mbh)-OH werden in 20 ml 90-proz. Trifluoressigsäure gelöst. Man läßt eine Stunde bei Raumtemperatur stehen, engt ein und verreibt den Rückstand mit Äther. Ausb. 2.11 g (92 %), Schmp. 70-78° (Zers.).

f) H-Arg-Lys-Tyr-Phe-Gln-OH-diacetat

2 g Z-Arg($Z_2$)-Lys-Tyr-Phe-Gln-OH werden in 90-proz. Essigsäure gelöst und analog Beispiel 1h katalytisch hydriert. Der Rückstand wird in 90-proz. Methanol an einem vernetzten hydroxypropylierten Dextrangel chromatographiert. Ausb. 473 mg. $[\alpha]_D^{21} = -7.4°$ (c = 1, Wasser) Aminosäureanalyse (Hydrolyse in 6N HCl, 24 Stunden bei 120°) : Glu 1.0, Tyr 0.91, Phe 0.99, Lys 1.05, Arg 0.94.

**Patentansprüche** (für die Vertragsstaaten : BE, CH, DE, FR, GB, IT, LI, NL, SE)

1. Peptid der Formel

$$B_1—B_2—A_1—A_2—X$$

in welcher bedeuten :

$B_1$ Arginin oder Lysin, jeweils in der L- oder D-Konfiguration, wobei die α-Aminogruppe gegebenenfalls acyliert ist,

$B_2$ Arginin oder Lysin,

$A_1$ Phenylalanin, Tyrosin oder Tryptophan, jeweils in der L- oder D-Konfiguration,

$A_2$ Phenylalanin, Tyrosin oder Tryptophan und

X Glutamin, Prolin, Glycin oder Arginin, wobei die Carboxylgruppe gegebenenfalls mit einem aliphatischen Alkohol mit 1-6 C-Atomen verestert ist,

sowie dessen physiologisch verträglichen Salze.

2. Peptid gemäß Anspruch 1, welches N-terminal eine freie Aminogruppe aufweist.

3. Peptid gemäß Anspruch 1, welches N-terminal mit einer Schutzgruppe vom Urethantyp geschützt ist.

4. Peptid H-Lys-Arg-Tyr-Tyr-Gly-OEt oder dessen physiologisch verträgliches Salz.

5. Peptid Y-Lys-Lys-Tyr-Phe-Arg-OH, worin Y H oder Z bedeutet, oder dessen physiologisch verträgliches Salz.

6. Peptid Y-D-Lys-Arg-D-Phe-Trp-Pro-OH, worin Y H oder Z bedeutet, oder dessen physiologisch verträgliches Salz.

7. Peptid H-Arg-Lys-Tyr-Phe-Gln-OH oder dessen physiologisch verträgliches Salz.

8. Verfahren zur Herstellung eines Peptids gemäß einem der Ansprüche 1-7, dadurch gekennzeichnet, daß man eine Aminosäuresequenz der Formel

$$B_1—B_2—A_1—A_2—X$$

nach den Methoden der Peptidchemie aufbaut und das erhaltene Peptid gegebenenfalls in ein physiologisch verträgliches Salz überführt.

9. Peptid gemäß einem der Ansprüche 1-7 zur Anwendung als Heilmittel.

10. Peptid gemäß einem der Ansprüche 1-7 zur Anwendung als Heilmittel bei der Behandlung von Immundefizienzen, viralen und fungoiden, sowie chronisch bakteriellen Infekten, Autoimmunkrankheiten, sowie zur Therapie von Erkrankungen, die durch Zellen mit immunologisch relevanten Veränderungen der Zellmembranmerkmale (z. B. Tumorzellen) verursacht werden.

11. Pharmazeutische Zubereitungen enthaltend ein Peptid gemäß einem der Ansprüche 1-7 oder dessen physiologisch verträgliches Salz.

12. Verfahren zur Herstellung einer Zubereitung gemäß Anspruch 11, dadurch gekennzeichnet, daß man ein Peptid gemäß einem der Ansprüche 1-7 oder dessen physiologisch verträgliches Salz zusammen mit den üblichen Zusatzstoffen in eine geeignete Darreichungsform bringt.

13. Verwendung eines Peptids gemäß einem der Ansprüche 1-7 zur Beeinflussung der Reifung von T-Lymphozyten.

**Patentansprüche** (für den Vertragsstaat AT)

1. Verfahren zur Herstellung eines Peptids der Formel

$$B_1—B_2—A_1—A_2—X$$

in welcher bedeuten :

$B_1$ Arginin oder Lysin, jeweils in der L- oder D-Konfiguration, wobei die $\alpha$-Aminogruppe gegebenenfalls acyliert ist,

$B_2$ Arginin oder Lysin,

$A_1$ Phenylalanin, Tyrosin oder Tryptophan, jeweils in der L- oder D-Konfiguration,

$A_2$ Phenylalanin, Tyrosin oder Tryptophan und

X Glutamin, Prolin, Glycin oder Arginin, wobei die Carboxylgruppe gegebenenfalls mit einem aliphatischen Alkohol mit 1-6 C-Atomen verestert ist,

oder dessen physiologisch verträglichen Salzen, dadurch gekennzeichnet, daß man eine Aminosäuresequenz der Formel

$$B_1—B_2—A_1—A_2—X$$

nach den Methoden der Peptidchemie aufbaut und das erhaltene Peptid gegebenenfalls in sein physiologisch verträgliches Salz überführt.

2. Verfahren gemäß Anspruch 1, dadurch gekennzeichnet, daß ein Peptid hergestellt wird, welches N-terminal eine freie Aminogruppe aufweist.

3. Verfahren gemäß Anspruch 1, dadurch gekennzeichnet, daß ein Peptid hergestellt wird, welches N-terminal mit einer Schutzgruppe vom Urethantyp geschützt ist.

4. Verfahren zur Herstellung eines Peptids H-Lys-Arg-Tyr-Tyr-Gly-OEt oder dessen physiologisch verträglichem Salz, dadurch gekennzeichnet, daß man diese Sequenz nach den Methoden der Peptidchemie aufbaut und das erhaltene Peptid gegebenenfalls in sein physiologisch verträgliches Salz überführt.

5. Verfahren zur Herstellung eines Peptids Y-Lys-Lys-Tyr-Phe-Arg-OH, worin Y H oder z bedeutet, oder dessen physiologisch verträglichem Salz, dadurch gekennzeichnet, daß man diese Sequenz nach den Methoden der Peptidchemie aufbaut und das erhaltene Peptid gegebenenfalls in sein physiologisch verträgliches Salz überführt.

6. Verfahren zur Herstellung eines Peptids Y-D-Lys-Arg-D-Phe-Trp-Pro-OH, worin Y H oder Z bedeutet oder dessen physiologisch verträglichem Salz, dadurch gekennzeichnet, daß man diese

Sequenz nach den Methoden der Peptidchemie aufbaut und das erhaltene Peptid gegebenenfalls in sein physiologisch verträgliches Salz überführt.

7. Verfahren zur Herstellung eines Peptids H-Arg-Lys-Tyr-Phe-Gln-OH oder dessen physiologisch verträglichem Salz, dadurch gekennzeichnet, daß man diese Sequenz nach den Methoden der Peptidchemie aufbaut und das erhaltene Peptid gegebenenfalls in sein physiologisch verträgliches Salz überführt.

8. Verfahren zur Herstellung eines Peptids gemäß einem der Ansprüche 1-7 zur Anwendung als Heilmittel.

9. Verfahren zur Herstellung eines Peptids gemäß einem der Ansprüche 1-7 zur Anwendung als Heilmittel bei der Behandlung von Immundefizienzen, viralen und fungoiden sowie chronisch bakteriellen Infekten, Autoimmunkrankheiten, sowie zur Therapie von Erkrankungen die durch Zellen mit immunologisch relevanten Veränderungen der Zellmembranmerkmale (z. B. Tumorzellen) verursacht werden.

10. Verfahren zur Herstellung einer pharmazeutischen Zubereitung enthaltend ein Peptid hergestellt gemäß einem der Ansprüche 1-7 oder dessen physiologisch verträgliches Salz, dadurch gekennzeichnet, daß man dieses Peptid oder dessen physiologisch verträgliches Salz zusammen mit den üblichen Zusatzstoffen in eine geeignete Darreichungsform bringt.

11. Verfahren zur Herstellung eines Peptids gemäß einem der Ansprüche 1-7 zur Verwendung als Mittel zur Beeinflussung der Reifung von T-Lymphozyten.

**Claims** (for the Contracting States : BE, CH, DE, FR, GB, IT, LI, NL, SE)

1. A peptide of the formula

$$B_1—B_2—A_1—A_2—X$$

in which :

$B_1$ denotes arginine or lysine, in each case in the L- or D-configuration, in which $\alpha$-amino group may be acylated,

$B_2$ denotes arginine or lysine,

$A_1$ denotes phenylalanine, tyrosine or tryptophan, in each case in the L- or D-configuration,

$A_2$ denotes phenylalanine, tyrosine or tryptophan and

X denotes glutamine, proline, glycine or arginine, in which the carboxyl group is, if desired, esterified with an aliphatic alcohol having 1 to 6 carbon atoms,

and physiologically acceptable salts thereof.

2. A peptide as claimed in claim 1 having an N-terminal free amino group.

3. A peptide as claimed in claim 1 being protected in the N-terminal position by a protective groupe of the urethane type.

4. A peptide H-Lys-Arg-Tyr-Gly-OEt or a physiologically acceptable salt thereof.

5. A peptide Y-Lys-Lys-Tyr-Phe-Arg-OH, wherein Y denotes H or Z or a physiologically acceptable salt thereof.

6. A peptide Y-D-Lys-Arg-D-Phe-Trp-Pro-OH, wherein Y denotes H or Z or a physiologically acceptable salt thereof.

7. A peptide H-Arg-Lys-Tyr-Phe-Gln-OH or a physiologically acceptable salt thereof.

8. A process for the preparation of a peptide as claimed in any one of claims 1 to 7, which comprises building up an aminoacid sequence of the formula

$$B_1—B_2—A_1—A_2—X$$

according to methods of peptide chemistry and optionally converting the peptide obtained into a physiologically acceptable salt.

9. A peptide as claimed in any one of claims 1 to 7 to be used as a medicament.

10. A peptide as claimed in any one of claims 1 to 7 to be used as a medicament in the treatment of deficiencies of immunity, of viral and fungoid and also chronic bacterial infections, autoimmunity diseases, and for the thereapy of diseases cuased by cells having immunologically relevant alterations in the characteristics of the cell membrane (for example tumor cells).

11. A pharmaceutical preparation containing a peptide as claimed in any one of claims 1 to 7 or physiologically acceptable salts thereof.

12. A process for the preparation of a composition as claimed in claim 11, which comprises converting a peptide as claimed in any one of claims 1 to 7 or a physiologically acceptable salt thereof together with the usual auxiliaries into a suitable form of administration.

13. The use of a peptide as claimed in any one of claims 1 to 7 for influencing the maturation of T-lymphocytes.

**Claims** (for the Contracting State AT)

1. A process for the preparation of a peptide of the formula

$$B_1—B_2—A_1—A_2—X$$

in which :

$B_1$ denotes arginine or lysine, in each case in the L- or D-configuration, in which the α-amino group is, if desired, acylated,

$B_2$ is arginine or lysine,

$A_1$ is phenylalanine, tyrosine or tryptophan, in each case in the L- or D-configuration,

$A_2$ is phenylalanine, tyrosine or tryptophan and

X is glutamine, proline, glycine or arginine, in which the carboxyl group, is, if desired, esterfied with an aliphatic alcohol having 1-6 carbon atoms,

or of the physiologically acceptable salts thereof, which comprises building up an aminoacid sequence of the formula

$$B_1—B_2—A_1—A_2—X$$

in accordance with methods of peptide chemistry and optionally converting the peptide obtained into its physiologically acceptable salt.

2. A process as claimed in claim 1, which comprises preparing a peptide having an N-terminal free amino group.

3. A process as claimed in claim 1, which comprises preparing a peptide which is protected by an N-terminal protective groupe of the urethane type.

4. A process for the preparation of a peptide H-Lys-Arg-Tyr-Tyr-Gly-OEt or of a physiologically acceptable salt thereof, which comprises building up this sequence in accordance with methods of peptide chemistry and optionally converting the peptide obtained into its physiologically acceptable salt.

5. A process for the preparation of a peptide Y-Lys-Lys-Tyr-Phe-Arg-OH in which Y denotes H or Z, or of its physiologically acceptable salt, which comprises building up this sequence in accordance with methods of peptide chemistry and optionally converting the peptide obtained into its physiologically acceptable salt.

6. A process for the preparation of a peptide Y-D-Lys-Arg-D-Phe-Trp-Pro-OH, in which Y denotes H or Z, or of its physiologically acceptable salt, which comprises building up this sequence in accordance with methods of peptide chemistry and optionally converting the peptide obtained into its physiologically acceptable salt.

7. A process for the preparation of a peptide H-Arg-Lys-Tyr-Phe-Gln-OH or of its physiologically acceptable salt, which comprises building up this sequence in accordance with methods of peptide chemistry and optionally converting the peptide obtained into its physiologically acceptable salt.

8. A process for the preparation of a peptide as claimed in any one of claims 1 to 7 to be used as a medicament.

9. A process for the preparation of a peptide as claimed in any one of claims 1 to 7 to be used as a medicament in the treatment of deficiencies of immunity, viral and fungoid and also chronic bacterial infections, autoimmunity diseases, and for the therapy of diseases caused by cells having immunology relevant alterations in the characteristics of the cell membrane (for example tumor cells).

10. A process for the preparation of a pharmaceutical composition containing a peptide prepared according to any one of claims 1 to 7 or a physiologically acceptable salt thereof, which comprises converting the peptide or its pharmaceutically acceptable salt together with the usual auxiliaries into a suitable form of administration.

11. A process for the preparation of a peptide as claimed in any one of claims 1 to 7 to be used as a medicament to influence the maturation of T-lymphocytes.

**Revendications** (pour les Etats contractants : BE, CH, DE, FR, GB, IT, LI, NL, SE)

1. Peptide de formule

$$B_1—B_2—A_1—A_2—X$$

dans laquelle :

$B_1$ représente l'arginine ou la lysine, chacune dans la configuration L ou D, le groupe α-amino étant éventuellement acylé,

$B_2$ représente l'arginine ou la lysine,

$A_1$ représente la phénylalanine, la tyrosine ou le tryptophane, chacun dans la configuration L ou D,

$A_2$ représente la phénylalanine, la tyrosine ou le tryptophane, et

X représente la glutamine, la proline, la glycine ou l'arginine, le groupe carboxy étant éventuellement estérifié avec un alcool aliphatique ayant de 1 à 6 atomes de carbone,
ainsi que les sels acceptables du point de vue physiologique de celui-ci.

2. Peptide selon la revendication 1, dont la terminaison azotée présente un groupe amino libre.

3. Peptide selon la revendication 1, dont la terminaison azotée est protégée avec un groupe protecteur du type uréthanne.

4. Peptide H-Lys-Arg-Tyr-Tyr-Gly-OEt ou sels acceptables du point de vue physiologique de celui-ci.

5. Peptide Y-Lys-Lys-Tyr-Phe-Arg-OH, dans lequel Y représente H ou Z, ou sels acceptables du point de vue physiologique de celui-ci.

6. Peptide Y-D-Lys-Arg-D-Phe-Trp-Pro-OH, dans lequel Y représente H ou Z, ou sels acceptables du point de vue physiologique de celui-ci.

7. Peptide H-Arg-Lys-Tyr-Phe-Gln-OH ou sels acceptables du point de vue physiologique de celui-ci.

8. Procédé pour la préparation d'un peptide selon l'une des revendications 1 à 7, caractérisé par le fait que l'on fait la synthèse d'une séquence d'amino-acides de formule

$$B_1—B_2—A_1—A_2—X$$

suivant les méthodes de la chimie des peptides, et que l'on convertit éventuellement le peptide obtenu en un sel acceptable du point de vue physiologique.

9. Peptide selon l'une des revendications 1 à 7 à utiliser en tant que médicament.

10. Peptide selon l'une des revendications 1 à 7 à utiliser en tant que médicament dans le traitement d'immunodéficiences, d'infections virales et fongoïdes de même que bactériennes chroniques, d'affections auto-immunes, ainsi que pour le traitement de maladies qui sont causées par des cellules ayant d'importantes altérations immunologiques des caractéristiques de la membrane cellulaire (cellules tumorales par exemple).

11. Préparation pharmaceutique contenant un peptide selon l'une des revendications 1 à 7 ou un sel acceptable du point de vue physiologique de celui-ci.

12. Procédé pour la fabrication d'une préparation selon la revendication 11, caractérisé par le fait que l'on met sous une forme de présentation appropriée un peptide selon l'une des revendications 1 à 7 ou un sel acceptable du point de vue physiologique de celui-ci, conjointement avec les additifs usuels.

13. Utilisation d'un peptide selon l'une des revendications 1 à 7 pour influencer la maturation de lymphocytes T.

**Revendications** (pour l'Etat contractant AT)

1. Procédé pour la préparation d'un peptide de formule

$$B_1—B_2—A_1—A_2—X$$

dans laquelle :

$B_1$ représente l'arginine ou la lysine, chacune dans la configuration L ou D, le groupe α-amino étant éventuellement acylé,

$B_2$ représente l'arginine ou la lysine,

$A_1$ représente la phénylalanine, la tyrosine ou le tryptophane, chacun dans la configuration L ou D,

$A_2$ représente la phénylalanine, la tyrosine ou le tryptophane, et

X représente la glutamine, la proline, la glycine ou l'arginine, le groupe carboxy étant éventuellement estérifié avec un alcool aliphatique ayant de 1 à 6 atomes de carbone,
ou les sels acceptables du point de vue physiologique de celui-ci, caractérisé par le fait que l'on fait la synthèse d'une séquence d'amino-acides de formule

$$B_1—B_2—A_1—A_2—X$$

suivant les méthodes de la chimie des peptides et que l'on convertit éventuellement le peptide obtenu en son sel acceptable du point de vue physiologique.

2. Procédé selon la revendication 1, caractérisé par le fait que l'on prépare un peptide dont la terminaison azotée présente un groupe amino libre.

3. Procédé selon la revendication 1, caractérisé par le fait que l'on prépare un peptide dont la terminaison azotée est protégée avec un groupe protecteur du type uréthanne.

4. Procédé pour la préparation d'un peptide H-Lys-Arg-Tyr-Tyr-Gly-OEt ou d'un sel acceptable du point de vue physiologique de celui-ci, caractérisé par le fait que l'on fait la synthèse de cette séquence suivant les méthodes de la chimie des peptides et que l'on convertit éventuellement le peptide obtenu en son sel acceptable du point de vue physiologique.

5. Procédé pour la préparation d'un peptide Y-Lys-Lys-Tyr-Phe-Arg-OH, dans lequel Y représente H ou Z, ou d'un sel acceptable du point de vue physiologique de celui-ci, caractérisé par le fait que l'on fait

la synthèse de cette séquence suivant les méthodes de la chimie des peptides et que l'on convertit éventuellement le peptide obtenu en son sel acceptable du point de vue physiologique.

6. Procédé pour la préparation d'un peptide Y-D-Lys-Arg-D-Phe-Trp-Pro-OH, dans lequel Y représente H ou Z, ou d'un sel acceptable du point de vue physiologique de celui-ci, caractérisé par le fait que l'on fait la synthèse de cette séquence suivant les méthodes de la chimie des peptides et que l'on convertit éventuellement le peptide obtenu en son sel acceptable du point de vue physiologique.

7. Procédé pour la préparation d'un peptide H-Arg-Lys-Tyr-Phe-Gln-OH ou d'un sel acceptable du point de vue physiologique de celui-ci, caractérisé par le fait que l'on fait la synthèse de cette séquence suivant les méthodes de la chimie des peptides et que l'on convertit éventuellement le peptide obtenu en son sel acceptable du point de vue physiologique.

8. Procédé pour la préparation d'un peptide selon l'une des revendications 1 à 7 à utiliser en tant que médicament.

9. Procédé pour la préparation d'un peptide selon l'une des revendications 1 à 7 à utiliser en tant que médicament dans le traitement d'immunodéficiences, d'infections virales et fongoïdes de même que bactériennes chroniques, d'affections auto-immunes, de même que pour le traitement de maladies qui sont causées par des cellules ayant d'importantes altérations immunologiques des caractéristiques de la membrane cellulaire (cellules tumorales par exemple).

10. Procédé pour la fabrication d'une préparation pharmaceutique contenant un peptide préparé selon l'une des revendications 1 à 7 ou un sel acceptable du point de vue physiologique de celui-ci, caractérisé par le fait que l'on met sous une forme de présentation appropriée ce peptide ou un sel acceptable du point de vue physiologique de celui-ci, conjointement avec les additifs usuels.

11. Procédé pour la préparation d'un peptide selon l'une des revendications 1 à 7 à utiliser en tant qu'agent pour influencer la maturation de lymphocytes T.